# EUROPÄISCHE PATENTSCHRIFT

(11) **EP 1 898 965 B1**
(45) Veröffentlichungstag und Bekanntmachung des Hinweises auf die Patenterteilung: **19.08.2009**
(21) Anmeldenummer: 06723371.8
(22) Anmeldetag: 11.03.2006
(51) Int. Cl.: A61L 9/01, A61L 9/12, A61L 9/05, A47L 15/44, C11D 3/50, C11D 17/04, C11D 1/72

(54) **VORRICHTUNG ZUR DOSIERTEN EINBRINGUNG EINES FESTEN DUFTSTOFFES WÄHREND DER SPÜL- UND TROCKNUNGSPHASE IN SPÜLMASCHINEN**
DEVICE FOR THE DOSED INTRODUCTION OF A SOLID FRAGRANCE DURING THE RINSING AND DRYING PHASES IN DISHWASHERS
DISPOSITIF POUR LIBERER DE MANIERE DOSEE UNE FRAGRANCE SOLIDE PENDANT LES PHASES DE LAVAGE ET DE SECHAGE D'UN LAVE-VAISSELLE

(30) Priorität: 30.05.2005 DE 102005025041
(43) Veröffentlichungstag der Anmeldung: 19.03.2008
(73) Patentinhaber: Bell Flavors & Fragrances Duft und Aroma GmbH, 04205 Leipzig/Militz (DE)
(72) Erfinder: HUPPERT, Hans-Jürgen, 04207 Leipzig (DE); BERRIDO, Colin, Bagshot, Surrey GU 19 5NU (GB)
(74) Vertreter: Bockhorni & Kollegen
(86) Internationale Anmeldenummer: PCT/EP2006/002259
(87) Internationale Veröffentlichungsnummer: WO 2006/128506

(56) Entgegenhaltungen:
- EP-A- 0 691 102
- WO-A-96/38638
- WO-A-20/04035721
- DE-A1- 3 832 885
- DE-A1- 10 236 610
- DE-A1- 19 532 542
- DE-A1- 19 836 857
- DE-U- 1 863 561
- DE-U- 1 988 975
- US-A- 3 272 899
- "RÖMPP CHEMIE LEXIKON, 9. ERWEITERTE AUFLAGE" 1990, GEORG THIEME VERLAG , STUTTGART, NEW YORK , XP002386120 PUNKT "EUCALYPTUSÖL" Seite 1264 - Seite 1265

## Beschreibung

Die vorliegende Erfindung betrifft eine Einrichtung zur Beduftung von Spülmaschinen während längerer Spülpausen und zur Verbesserung des Abperlverhaltens des Spülwassers von den gespülten Gegenständen während der Trocknungsphase.

Laut Kundenbefragungen wird in Ein- oder Zweipersonenhaushalten die Spülmaschine nur zweimal pro Woche benutzt. Dies ist darauf zurückzuführen, dass sich nur in diesen Zeitabständen genügend zu reinigende Gegenstände für eine vollständige oder nahezu vollständige Auslastung der Spülmaschine angesammelt haben. Somit füllt sich in einem Zeitraum von zwei, drei oder mehr Tagen die Spülmaschine mit Gegenständen, die von Speiseresten verunreinigt sind. Dies führt bereits nach kurzer Zeit zur Entwicklung von unangenehmen Gerüchen in der Spülmaschine, welchen die Benutzer ausgesetzt sind, wenn sie die Spülmaschine öffnen, um weitere verunreinigte Gegenstände einzubringen. Es wird geschätzt, dass die Benutzer während der mehrtägigen Spülpausen ca. 8 bis 10-mal diesen Gerüchen ausgesetzt sind.

Geruchsverbessernde Mittel zur Beduftung von Spülmaschinen sind aus dem Stand der Technik bekannt. So stellen die Firmen Reckitt Benkiser und Henkel unter den Marken "Calgonite" und "Somat Deoperls" entsprechende Produkte her. Das Produkt der Firma Reckitt Benkiser basiert auf einer flüssigen Duftstoffmischung mit einem Gehalt von bis zu 60 % Duftstoff. Diese ist in einem Kunststoffbehälter gespeichert und weist eine Membran auf, durch welche der Duftstoff in das Innere der Spülmaschine diffundiert. Dieses Produkt hat den Nachteil, dass große Duftstoffmengen in dem Behälter erforderlich sind, um eine angemessene Wirkungsdauer von z. B. 30 Tagen zu sichern. Dies ist auf eine hohe Abgabe von flüssigem Duftstoff bei jedem Spülvorgang durch die Temperaturen des heißen Wassers und der heißen Luft während der Spül- und Trocknungsphasen zurückzuführen, Durch die übermäßige Duftstoffabgabe werden hohe Kosten verursacht. Außerdem erfolgt bei diesem Produkt die Duftstofffreisetzung im Verlaufe der Anwendungszeit des Produktes ungleichmäßig, d. h. es werden zunächst die leichtflüchtigeren Duftstoffbestandteile freigesetzt. Dadurch ergibt sich im Verlauf der Anwendungszeit eine beträchtliche Veränderung des Duftes im Vergleich zum ursprünglichen Duft, was für die Akzeptanz dieses Mittels bei den Verbrauchtern nicht förderlich ist.

Das Produkt der Firma Henkel basiert auf dem Freisetzen von Duftstoff aus einem granulierten festen Trägermaterial von Ethylvinylacetat (EVA), einem Stoff mit begrenzter Wasserlöslichkeit, welcher in DE 10237066 beschrieben ist. Der Duftstoffanteil, welcher in dieses Trägermaterial eingebracht werden kann, beträgt maximal 40 %. Für die Freisetzung des Duftes in der Spülmaschine wird ein spezieller Behälter verwendet, welcher in DE 10303352 beschrieben ist, um das Ausströmen von Duftstoff bei Kontakt mit heißem Wasser und heißer Luft während der Spül- und Trocknungsphasen der Spülmaschine zu sichern. Dies geschieht durch die hohen Wasser- und Lufttemperaturen, die während der Spül-und Trocknungsphasen in der Spülmaschine auftreten. Bei diesem Produkt ist die Duftstoffabgabe jedoch beschränkt. Der wesentlichste Nachteil bei diesem Mittel besteht darin, dass ein maximaler Duftstoffgehalt von 40 % die Verwendung großer Mengen an Trägermaterial erfordert, um eine ausreichende Duftintensität im Inneren der Spülmaschine während der Spülpausen aufrecht zu erhalten.

Ein weiteres Problem, das bei der Benutzung von Spülmaschinen auftritt, besteht darin, dass "Schleier" oder Flecken auf den Gegenständen nach dem Spülen zurückbleiben, was sich besonders bei Glasgegenständen bemerkbar macht. Dieses Problem ist in Gegenden besonders akut, in welchen ein hoher Mineraliengehalt im Trinkwasser vorhanden ist. Dieses Problem wurde bisher dadurch gelöst, dass neben dem Spülmittel und gegebenenfalls einem Mittel zur Geruchsverbesserung ein weiteres, das Abperlen des an den gespülten Gegenständen haftenden Spülwassers verbesserndes Mittel, ein so genanntes Crlanzspülmittel zusätzlich in die Spülmaschine eingebracht wurde. Ein Beispiel für ein solches handelsübliches Produkt ist das unter dem Namen "Finish" von der Firma Reckitt Benkiser hergestellte Erzeugnis.

Ein großer Nachteil der bekannten Mittel zur Geruchsverbesserung sowie der Glanzspülmittel besteht darin, dass diese Produkte zusätzlich zu dem eigentlichen Spülmittel als Einzelprodukte in die Spülmaschine eingegeben werden müssen.

Es ist deshalb Aufgabe der Erfindung, eine Einrichtung zu entwickeln, mit welcher die Beduftung der Spülmaschine während längerer Spülpausen sowie gleichzeitig eine Verbesserung des Abperlverhaltens des Spülwassers von den gespülten Gegenständen und damit ein Glanzeffekt ermöglicht wird.

Die Aufgabe wird durch die Merkmale der Patentansprüche 1 oder 2 oder 3 gelöst.

Vorteilhafte Ausführungsformen der Erfindung bilden die Merkmale der Patentansprüche 4 bis 8.

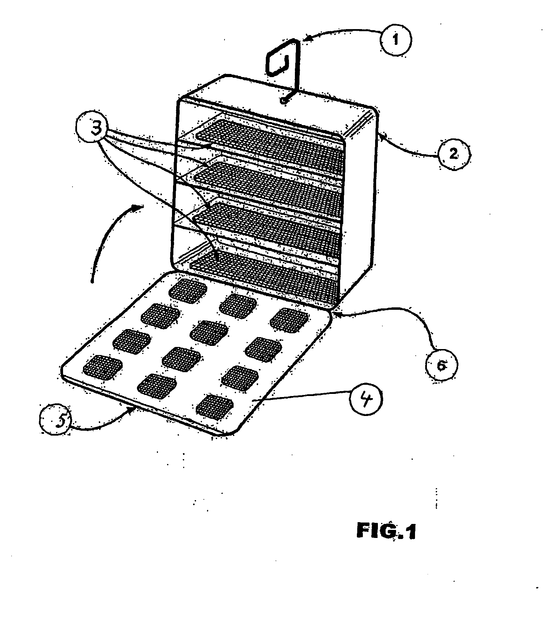

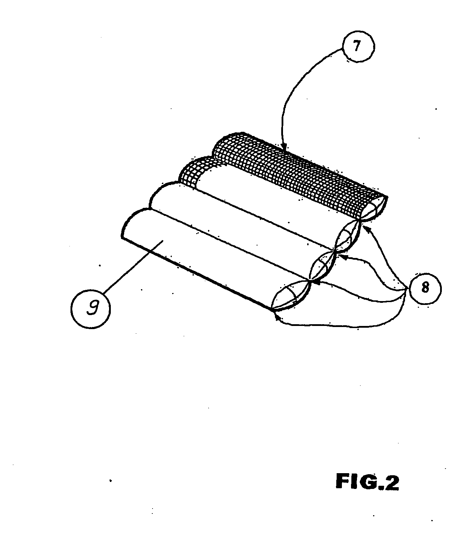

Die Erfindung soll nachfolgend anhand eines Ausführungsbeispieles unter Bezugnahme auf die Zeichnungen der Figuren 1 und 2 näher erläutert werden. Der in der erfindungsgemäßen Einrichtung verwendete an sich bekannte feste Duftstoff umfasst ein bei Normaltemperatur festes Trägermaterial, das aus Fettalkohol besteht oder einen Anteil von Fettalkohol besitzt, und 40 bis 60 Gew.-% des festen Duftstoffes ausmacht, und einen darin gelösten flüssigen Duftstoff mit einem Gewichtsanteil von 60 bis 40 %. Während der Spül- und Trocknungsphasen, bei welchen der feste Duftstoff mit heißem Wasser bzw. heißer Luft bei Temperaturen von über 50 °C beaufschlagt wird, erfolgt eine Freisetzung von Duftstoff aus dem festen Trägermaterial zur Beduftung des Innenraumes der Spülmaschine. Gleichzeitig erfolgt während der Spülphasen, bei denen das feste Trägermaterial mit heißem Wasser in Berührung kommt, eine Lösung von jeweils geringen Mengen an Fettalkohol in heißem Wasser, und der gelöste Fettalkohol sorgt für eine effektive Verbesserung des Abperlvermögens des Wassers von den gespülten Gegenständen. Dadurch wird eine Schleier- bzw. Fleckbildung auf den gespülten Gegenständen, die sich insbesondere auf Glasgegenständen bemerkbar macht, vermieden. Die Oberflächen der gespülten Gegenstände zeigen dadurch nach dem Abtrocknen einen sichtbaren Glanz.

Die Aufnahme von flüssigem Duftstoff in einem festen Trägermaterial aus Fettalkohol oder aus einem Gemisch von Fettalkohol und anderen Substanzen ist in dem Europäischen Patent EP 1 549 729 sowie in der deutschen Patentanmeldung DE 102 47 583.0 beschrieben. Überraschend hat sich gezeigt, dass das für die Aufnahme des flüssigen Duftstoffes gewählte Trägermaterial in Form von Fettalkohol oder einem Gemisch von Fettalkohol und anderen Substanzen durch die Lösung von Fettalkohol im Spülwasser zusätzlich einen für das Abperlverhalten des Wassers beim Trocknungsprozess günstigen Einfluss ausübt.

Als besonders geeignetes Mittel hat sich eine Lösung von Duftstoff in Fettalkohol C22 erwiesen.

Darüber hinaus wurde ermittelt, dass bei Ersatz von bis zu 20 Gew.-% Fettalkohol durch von Fettalkohol-Ethylen/Propylen-Oxid abgeleitete oberflächenaktive Stoffe eine weitere Verbesserung des Abperlverllaltens des Spülwassers von den gespülten Gegenständen erreicht werden kann, ohne dass eine Verminderung der Aufnahmefähigkeit für Duftstoff im Trägermaterial auftritt. Ein Beispiel für ein handelsübliches Produkt, welches vorstehend genannte Zusammensetzung aufweist, und bis zu 20 % des Trägermaterials ersetzen kann, ist DEHYPON 3697 von der Firma Cognis-Care Chemicals. Obwohl bereits die Wahl von Fettalkohol oder von Stoffen mit einem Anteil an Fettalkohol, insbesondere von Fettalkohol C22 als Trägermaterial für den Duft in überraschender Weise für ein gutes Abperlverhalten des Wassers und damit für eine verminderte Schleier- und Fleckbildung auf den Gegenständen und damit für eine Glanzbildung sorgt, wird dieser Effekt durch den vorgenannten Zusatz von oberflächenaktiven Stoffen, die von Fettalkohol-Ethylen/Propylen-Oxid abgeleitet sind, weiter verstärkt.

Zur effizienten Freisetzung sowohl des Duftstoffes als auch der das Abperlverhalten des Spülwassers verbessernden Substanzen dient ein Korpus, welcher innerhalb der Spülmaschine befestigt wird. Dieser sichert, dass ein guter Kontakt des festen Duftstoffes mit dem Wasser während der Spülphasen zustande kommt und verhindert, dass der in der Einrichtung eingeschlossene feste Duftstoff unter seinem eigenen Gewicht bei Temperaturen von mehr als 50 °C, welche während der Spül- und Trocknungsphasen erreicht werden, agglomerieren kann. Wenn das Agglomerieren nicht verhindert wird, könnte eine ausreichende Freisetzung von Duft und von den das Abperlverhalten des Spülwassers verbessernden Stoffen im Anwendungszeitraum mehr und mehr beeinträchtigt werden.

Figur 1 zeigt eine Ausführungsform einer erfindungsgemäßen Einrichtung. Sie umfasst eine durchströmbare, nachfüllbare Einheit, welche den festen, körnigen, das Abperlverhalten des Wassers verbessernden festen Duftstoff aufnimmt.

Figur 2 zeigt eine weitere Form einer erfindungsgemäßen Einrichtung. Diese kann, mit einer Aufhängung versehen, selbständig an geeigneter Stelle in den Innenraum der Spülmaschine eingehängt werden, oder sie kann in einem der Figur 1 ähnlichen Korpus, der jedoch keine Zwischenböden aufweist, als Nachfüllbeutel für diese Einrichtung verwendet werden.

Die Einrichtung nach Figur 1 umfasst im Wesentlichen einen Haken 1, welcher die hängende Befestigung der Vorrichtung z. B. an einem der Spülkörbe innerhalb der Spülmaschine ermöglicht. Sie umfasst weiterhin einen starren Korpus 2 mit siebartig durchströmbarer Rückwand (in Fig. 1 nicht sichtbar), welcher den körnigen festen Duftstoff auf einer Anzahl von siebartigen Zwischenböden 3 aufnimmt. Der körnige, als Chips, Perlen oder Granulat vorliegende feste Duftstoff wird vorzugsweise einschichtig auf jeden der Böden 3 aufgebracht. Die Einrichtung umfasst außerdem eine siebartig durchströmbare Klappe 4, welche um ein Gelenk 6 verschwenkbar ist und durch eine mit dem Korpus 3 selbstverrastende Struktur 5, die in Figur 1 im Einzelnen nicht dargestellt ist, fest aber lösbar mit dem Korpus 3 verbunden wird.

Während des Spülphasen strömt Spülwasser durch die siebartigen Durchbrüche der Klappe 4 und der Rückwand des Korpus 2 und löst den das Abperlverhalten des Spülwassers verbessernden Stoff, d. h. den als Trägermaterial für den flüssigen Duftstoff dienenden Fettalkohol bzw. eventuell mit diesem vermischte Aktivstoffe. Gleichzeitig wird bei Einwirkung von Wärme, d. h. von Warmwasser und vor allem von der nach den Spülphasen einströmenden warmen Trocknungsluft, der Duftstoff aus der festen körnigen Struktur freigesetzt.

Der im Spülwasser gelöste Anteil von Fettalkohol und von eventuell weiteren oberflächenaktiven Stoffen sorgt für ein weitgehend "schleier"- und fleckloses Abperlen des Spülwassers im. Trocknungsprozess, und der aus dem körnigen festen Duftstoff durch die Trocknungswärme freigesetzte Duft sorgt für einen über Tage anhaltenden Wohlgeruch im Inneren der Spülmaschine, welcher die unangenehmen Gerüche der mit Speiseresten verunreinigten Gegenstände, die sich über mehrere Tage in der Spülmaschine ansammeln, weitestgehend überdeckt.

Bei Verwendung von Duftstoffen, welche gleichzeitig eine antibakterielle Wirkung entfalten, wie z. B. von Cineol oder Methyl-Phenyl-Butanol, wird dem erfindungsgemäßen Mittel auch noch eine desodorierende Wirkung verliehen, so dass die unangenehmen Gerüche nicht nur überdeckt sondern zusätzlich noch bekämpft werden. Somit ist es erfindungsgemäß möglich, dem Mittel, dem bei Produkten des Standes der Technik lediglich eine beduftende Rolle zufiel, eine dreifache Wirkung zu verleihen, ohne dass es notwendig ist, spezielle Glanzspüler oder desodorierende Substanzen zuzusetzen.

Figur 2 zeigt eine alternative Ausführungsform einer Einrichtung zur Beduftung von Spülmaschinen in den Spül- und Trocknungsphasen, welche ebenfalls dafür sorgt, dass der körnige feste Duftstoff durch die Beaufschlagung mit Temperaturen von über 50 °C während der Spül- und Trocknungsphasen im Inneren der Spülmaschine nicht agglomerieren kann.

Diese Vorrichtung ist als Beutel aus halbweichem siebartig durchbrochenem thermoplastischen Kunststoff ausgebildet, und in Abständen durch nahtartige querverlaufende Heißsiegelungen 8 in mehrere Kammern 9 eingeteilt. Weil auch hier, ähnlich wie durch die Zwischenböden 3 in der Vorrichtung nach Figur 1, nur kleine Mengen des körnigen Mittels in einer Kammer liegen, ist die Gefahr, dass die Körner bei Einwirkung von Wärme über 50 °C unter Einwirkung ihres eigenen Gewichtes agglomerieren, praktisch nicht gegeben.

Die in Figur 2 dargestellte Ausführungsform der Einrichtung kann auch als Nachfülleinheit für einen der Figur 1 ähnlichen Korpus genutzt werden, der die in Figur 1 vorgesehenen Zwischenboden 3 nicht enthält.

## Patentansprüche

1. Einrichtung zur Beduftung von Spülmaschinen während längerer Spülpausen und zur gleichzeitigen Verbesserung des Abperlverhaltens des Spülwassers in der Trocknungsphase, umfassend einen in die Spülmaschine einhängbaren, aus starrem Material gefertigten Korpus (2) mit einer in Hängeposition senkrechten, siebartig durchströmbaren Rückwand und einer Anzahl von im Inneren des Korpus (2) in Abständen waagerecht angeordneten, siebartig durchströmbaren Zwischenböden (3) sowie einer mit dem Korpus (2) lösbar verbundenen siebartig durchströmbaren senkrechten Vorderwand (4), wobei
ein an sich bekannter fester Duftstoff in den Korpus (2) eingebracht ist, welcher ein bei Normaltemperatur festes Trägermaterial aus Fettalkohol oder einem fettalkoholhaltigen Gemisch und einen darin gelösten, bei Normaltemperatur flüssigen Duftstoff in einem Mengenverhältnis umfasst, welhes zwischen 40 Gew.-% und 60 Gew.-% Trägermaterial und zwischen 60 Gew.-% und 40 Gew.-% Duftstoff enthält.

2. Einrichtung zur Beduftung von Spülmaschine während längerer Spülpausen und zur gleichzeitigen Verbesserung des Abperlverhaltens des Spülwassers in der Trocknungsphase, umfassend einen als Beutel aus halbweichem siebartig durchbrochenem thermoplastischen Kunststoff ausgebildeten Korpus (7), der durch in Abständen querverlaufende nahtartige Heißversiegelungen (8) in mehrere Kammern (9) unterteilt ist, wobei
ein an sich bekannter fester Duftstoff in die Kammern (9) des Korpus (7) eingebracht ist, welcher bei Normaltemperatur festes Trägermaterial aus Fettalkohol oder einem fettalkoholartigen Gemisch und einen darin gelösten, bei Normaltemperatur flüssigen Duftstoff in einem Mengenverhältnis umfasst, welches zwischen 40 Gew.-% und 60 Gew.-% Trägermaterial und zwischen 60 Gew.-% und 40 Gew.-% Duftstoff enthält.

3. Einrichtung zur Beduftung von Spülmaschinen während längerer Spülpausen und zur gleichzeitigen Verbesserung des Abperlverhaltens des Spülwassers in der Trocknungsphase, umfassend einen in die Spülmaschine einhängbaren, aus starrem Material gefertigten Korpus mit einer in Hängeposition senkrechten, siebartig durchströmbaren Rückwand und einer mit dem Korpus lösbar verbundenen siebartig durchströmbaren senkrechten Vorderwand, wobei
der Korpus mit dem an sich bekannten festen Duftstoff gefüllten Korpus (7) nach Anspruch 2 als Nachfülleinheit bestückt ist.

4. Einrichtung nach einem der Ansprüche 1 bis 3,
**dadurch gekennzeichnet**
**dass** als Fettalkohol zumindest teilweise Fettalkohol C22 verwendet wird.

5. Einrichtung nach mindestens einem der vorhergehenden Ansprüche
**dadurch gekennzeichnet,**
**dass** bis zu 20 Gew.-% des Trägermateriales oberflächenaktive Stoffe sind, die von Fettalkohol-Ethylen-/Propylen-Oxid abgeleitet sind.

6. Einrichtung nach mindestens einem der Ansprüche 1 bis 4,
**dadurch gekennzeichnet,**
**dass** bis zu 20 Gew.-% des Trägermaterials modifizierte Fettalkohol-Polyglycol-Ether sind.

7. Einrichtung nach mindestens einem der vorhergehenden Ansprüche,
**dadurch gekennzeichnet, dass** der Duftstoff zusätzlich einen antibakteriellen Effekt ausweist.

8. Einrichtung nach Anspruch 7,
**dadurch gekennzeichnet,**
**dass** als Duftstoff Cineol oder Methyl-Phenyl-Butanol verwendet wird.

## Claims

1. Device for fragrancing automatic dishwashers during protracted operating intervals and for simultaneously improving the pearl-off performance of the rinse water during the drying cycle, comprising a body (2), which is made of a rigid material and can be suspended in the dishwasher, and which has, in the suspended position, a vertical, sieve-like, permeable rear wall and a number of horizontal, sieve-like, permeable partitions (3) arranged at intervals inside the body (2), as well as a vertical, sieve-like, permeable front wall (4), which is detachably connected with the body (2), wherein a solid fragrance, known per se, is introduced into the body (2), which solid fragrance comprises a carrier material of fatty alcohols or a mixture containing fatty alcohols, said carrier material being solid at normal temperatures, and, dissolved in said carrier material, a fragrance that is liquid at normal temperatures, the solution being composed quantitatively of between 40 and 60 % by weight of carrier material and between 60 and 40 % by weight of fragrance.

2. Device for fragrancing automatic dishwashers during protracted operating intervals and for simultaneously improving the pearl-off performance of the rinse water during the drying cycle, comprising a body (7) configured as a pouch made of a sieve-like, perforated semi-soft thermoplastic, which is subdivided into several chambers (9) by spaced, transverse, seam-like heat seals (8), wherein a solid fragrance, known per se, is introduced into the chambers (9) of the body (7), which solid fragrance comprises a carrier material of fatty alcohols or a mixture containing fatty alcohols, said carrier material being solid at normal temperatures, and, dissolved in said carrier material, a fragrance that is liquid at normal temperatures, the solution being composed quantitatively of between 40 and 60 % by weight of carrier material and between 60 and 40 % by weight of fragrance.

3. Device for fragrancing automatic dishwashers during protracted operating intervals and for simultaneously improving the pearl-off performance of the rinse water during the drying cycle, comprising a body which is made of a rigid material and can be suspended in the dishwasher, and which has, in the suspended position, a vertical, sieve-like, permeable rear wall and a vertical, sieve-like, permeable front wall which is detachably connected with the body, wherein the body contains a refill unit in the form of the body (7) of claim 2, which is filled with solid fragrance known per se.

4. Device according to one of claims 1 to 3,
**characterised in that** the fatty alcohol used is at least partially C22 fatty alcohol.

5. Device according to at least one of the preceding claims,
**characterised in that** up to 20 wt. % of the carrier material are surfactants derived from fatty alcohol ethylene/propylene oxide.

6. Device according to at least one of claims 1 to 4,
**characterised in that** up to 20 wt. % of the carrier material are modified fatty alcohol polyglycol ethers.

7. Device according to at least one of the preceding claims,
**characterised in that** the fragrance has an additional, antibacterial effect

8. Device according to claim 7,
**characterised in that** cineole or methyl phenyl butanol is used as fragrance.

## Revendications

1. Dispositif pour parfumer des lave-vaisselles pendant des pauses de lavage prolongées et pour améliorer de manière simultanée le comportement de coulée de l'eau de rinçage dans la phase de séchage, comprenant un corps (2) fabriqué en matériau rigide, pouvant être accroché dans le lave-vaisselle avec une paroi arrière pouvant être traversée comme un tamis, perpendiculaire dans la position suspendue et un nombre de fonds intermédiaires (3) pouvant être traversés comme un tamis, disposés à intervalles horizontalement à l'intérieur du corps (2) ainsi qu'une paroi avant (4) perpendiculaire, pouvant être traversée comme un tamis, reliée de manière détachable au corps (2), une fragrance solide connue en soi étant introduite dans le corps (2), lequel comporte un matériau porteur solide à température normale composé d'alcool gras ou d'un mélange contenant de l'alcool gras et une fragrance dissoute dedans, liquide à température normale dans un rapport de quantité qui contient entre 40 et 60 % en poids de matériau porteur et entre 60 et 40 % en poids de fragrance.

2. Dispositif pour parfumer des lave-vaisselles pendant des pauses de lavage prolongées et pour améliorer de manière simultanée le comportement de coulée de l'eau de rinçage dans la phase de séchage, comprenant un corps (7) réalisé comme un sachet en matière thermoplastique, percé comme un tamis, demi-mou qui est divisé par des scellements thermiques (8) de type soudure, s'étendant transversalement à intervalles en plusieurs chambres (9), une fragrance solide connue en soi étant introduite dans les chambres (9) du corps (7), lequel comporte un matériau porteur solide à température normale composé d'alcool gras ou d'un mélange contenant de l'alcool gras et une fragrance dissoute dedans, liquide à température normale dans un rapport de quantité qui contient entre 40 et 60 % en poids de matériau porteur et entre 60 et 40 % en poids de fragrance.

3. Dispositif pour parfumer des lave-vaisselles pendant des pauses de lavage prolongées et pour améliorer de manière simultanée le comportement de coulée de l'eau de rinçage dans la phase de séchage, comprenant un corps fabriqué en matériau rigide, pouvant être accroché dans le lave-vaisselle avec une paroi arrière pouvant être traversée comme un tamis, perpendiculaire dans la position suspendue et une paroi avant perpendiculaire, pouvant être traversée comme un tamis, reliée de manière détachable au corps, le corps étant équipé du corps (7) rempli de la fragrance solide connue en soi selon la revendication 2 comme unité de recharge.

4. Dispositif selon l'une quelconque des revendications 1 à 3,
**caractérisé en ce que** de l'alcool gras C22 est utilisé au moins partiellement comme alcool gras.

5. Dispositif selon au moins l'une quelconque des revendications précédentes,
**caractérisé en ce que** jusqu'à 20 % en poids de matériau porteur sont des substances actives en surface qui sont dérivées d'oxyde de propylène/d'éthylène d'alcool gras.

6. Dispositif selon au moins l'une quelconque des revendications 1 à 4,
**caractérisé en ce que** jusqu'à 20 % en poids du matériau porteur sont des éthers de polyglycol d'alcool gras modifiés.

7. Dispositif selon au moins l'une quelconque des revendications précédentes,
**caractérisé en ce que** la fragrance présente en outre un effet antibactérien.

8. Dispositif selon la revendication 7,
**caractérisé en ce que** du cinéol ou du méthylphénylbutanol est utilisé comme fragrance.
